# EUROPÄISCHE PATENTANMELDUNG

(11) **EP 1 086 686 A1**
(43) Veröffentlichungstag der Anmeldung: **28.03.2001**
(21) Anmeldenummer: 00119156.8
(22) Anmeldetag: 05.09.2000
(51) Int. Cl.: A61K 7/48, A61K 7/06, C08F 290/10

(54) **Kosmetische Mittel mit einem Gehalt an Saccharidpolymeren oder Saccharidpolymerlatices**

(30) Priorität: 21.09.1999 DE 19945235
(71) Anmelder: Wella Aktiengesellschaft, 64274 Darmstadt (DE)
(72) Erfinder: Yaacoub, E.-J., 38159 Vechelde (DE); Koch, Ulrich, 38108 Braunschweig (DE); Allwohn, Jürgen, 65558 Burgschwalbach (DE); Birkel, Susanne, 95496 Glashütten (DE); Lede, Michael, 63225 Langen (DE)

(57) **Zusammenfassung**

Es werden kosmetische Mittel beschrieben mit einem Gehalt an mindestens einem Polymer, welches aus mindestens einer Monomerart (A) aufgebaut ist, welche ein mit einer radikalisch polymerisierbaren Gruppe substituiertes Kohlenhydrat oder Kohlenhydratderivat ist und eine oder mehrere Schutzgruppen enthalten kann in einer geeigneten kosmetischen Grundlage. Das Polymer wird vorzugsweise in Form eines durch Emulsionspolymerisation hergestellten Saccharidlatex eingesetzt.

## Beschreibung

Gegenstand der vorliegenden Erfindung sind kosmetische Mittel mit einem Gehalt an mindestens einem Polymer, welches aus mindestens einer Monomerart (A) aufgebaut ist, die ausgewählt ist aus mit einer radikalisch polymerisierbaren Gruppe substituierten Kohlenhydraten oder Kohlenhydratderivaten in einer geeigneten kosmetischen Grundlage. Das Polymer wird vorzugsweise in Form eines durch Emulsionspolymerisation hergestellten Saccharidlatex eingesetzt.

Ein ansprechendes äußeres Erscheinungsbild wurde schon immer als sehr wichtig angesehen. Eine besondere Rolle spielen dabei Frisur, Haut, Fingernägel, Augenbrauen etc.. Zur Verbesserung des Erscheinungsbildes werden Körperpflegeprodukte verwendet. Diesen Mitteln ist gemeinsam, daß sie in der Regel aus einer Vielzahl an Einzelsubstanzen oder Komponenten bestehen, die die unterschiedlichsten Aufgaben innerhalb der Rezeptur erfüllen. Zu den vielseitigsten Inhaltsstoffen von kosmetischen Produkten mit den unterschiedlichsten Funktionen und Wirkungen gehören die Polymere. Polymere können beispielsweise verdickend, konservierend, filmbildend, haarfestigend, haarpflegend oder konditionierend auf Haut und Haare wirken. Sie können die Produktkonsistenz positiv beeinflussen, den Transport und die Haftung von Wirkstoffen auf dem jeweiligen Substrat (z.B. Haut, Haare, Nägel) verbessern oder die Eigenschaften der übrigen Inhaltsstoffe vorteilhaft modifizieren.

Es besteht ein ständiger Bedarf an neuen Polymeren mit verbesserten Eigenschaften und guter Kompatibilität mit mit möglichst einer Viehlzahl von anderen, üblicherweise verwendeten kosmetischen Imnhaltstoffen. Von besonderem Vorteil sind Polymere, die auf der Basis von nachwachsenden Rohstoffen beruhen und die gut biologisch abbaubar sind. Die Aufgabe dieser Erfindung bestand also darin, neue Polymere zu finden, die eine oder mehrere dieser Funktionen erfüllen.

Eine solche Klasse von Polymeren wurde mit den unten näher definierten Saccharidpolymeren, insbesondere in Form von Saccharidpolymerlatices, gefunden. Diese sind in einer Vielzahl von verschiedenen kosmetischen Mitteln vorteilhaft einsetzbar. Gegenstand der Erfindung ist daher ein kosmetisches Mittel mit einem Gehalt an mindestens einem Polymer, welches aus mindestens einer Monomerart (A) aufgebaut ist, welche eine mit mindestens einer radikalisch polymerisierbaren Gruppe substituierte Verbindung ist, die ausgewählt ist aus Kohlenhydraten und Kohlenhydratderivaten und wobei die Verbindung eine oder mehrere Schutzgruppen enthalten kann, in einer geeigneten kosmetischen Grundlage. Die kosmetischen Mittel können in Form einer Lotion, einer Sprühlotion, einer Creme, eines Gels, eines Aerosol- oder Nonaerosol-Sprays, eines Aerosol- oder Nonaerosol-Schaums, eines Shampoos, einer Mascara, eines Make-ups, eines Lippenstifts oder eines Deodorans konfektioniert werden. Die erfindungsgemäß einzusetzenden Saccharidpolymere sind vorzugsweise in einer Menge von 0,01 bis 25, besonders bevorzugt von 0,1 bis 10 Gew.% enthalten. Vorzugsweise werden die Saccharidpolymere in Form eines Polymerlatex, insbesondere in Form eines der unten näher beschriebenen, durch Emulsionspolymerisation hergestellten Latices und Kern/Schale-Emulsionspolymerisaten eingesetzt.

Durch Emulsionspolymerisation erhältliche wässrige Polymerdispersionen (Latex) sind allgemein bekannt. Es handelt sich um fluide Systeme, die als disperse Phase Polymerteilchen und als kontinuierliche Phase Wasser oder ein wässriges Medium enthalten. Der Durchmesser der Polymerisatteilchen liegt typischerweise bei 0,01 bis 5 µm, insbesondere bei 0,01 bis 1 µm. Der Festkörperanteil kann dabei in der Regel bis zu 60% betragen, ohne daß das System hochviskos wird. Zur Stabilisierung sind in der Regel bis zu 10% Tenside und/oder Schutzkolloide enthalten. Die Tenside sind meistens anionisch, können aber auch kationisch oder nichtionisch sein. Aus J. Klein et al., *Makromol*. *Chem*., *Rapid Commun*. 6, 675-678, 1985 ist bekannt, 3-Methacryloyl-diisopropyliden-glucose in Emulsion zu polymerisieren, dann die Homopolymerisate auszufällen und chemisch zu entschützen, um wasserlösliche Polymerisate mit im Vergleich zu durch Lösungspolymerisation erhaltenen Polymeren höherer Molmasse und höherer Viskosität zu erzielen.

Für den Einsatz in den erfindungsgemäßen kosmetischen Mittel geeignet ist ein Polymerlatex, enthaltend mindestens ein in Wasser dispergiertes, durch Emulsionspolymerisation hergestelltes Polymer, dessen in disperser Verteilung befindliches Polymerisat in radikalisch polymerisierter Form aufgebaut ist aus mindestens einer Monomerart (A), welche eine mit mindestens einer radikalisch polymerisierbaren Gruppe substituiertes Kohlenhydrat oder Kohlenhydratderivat ist, welche vorzugsweise ausgewählt ist aus Sacchariden, Saccharidderivaten und Zuckeralkoholen, wobei die Monomerart (A) eine oder mehrere Schutzgruppen enthalten kann.

In einer bevorzugten Ausführungsform ist das Latexpolymer aufgebaut aus mindestens einer zweiten, von (A) verschiedenen, mit (A) radikalisch copolymerisierbaren Monomerart (B).

Die erfindungsgemäßen Polymerlatices sind dadurch charakterisiert, daß die Kohlenhydratkomponenten ihnen ihre spezifischen Qualitäten aufprägen. Zu diesen zählen Hydrophilie bzw. eine charakteristische Balance zwischen Hydrophilie und Hydrophobie, Kompatibilität insbesondere mit biologischen Systemen, und hier z.B. speziell mit der Haut, keine oder geringe Toxizität, biologische Abbaubarkeit usw..

In einigen Fällen kann es wegen der Eigenschaften der entstehenden Polymerlatices interessant sein,
- zwei der unter (A) angegebenen Monomerarten oder
- zwei Zuckermonomere (A) und ein Monomer der unter (B) angegebenen Verbindungen oder
- ein Zuckermonomer (A) und zwei bzw. drei Monomere (B) einzusetzen.

Die Monomerart (A) ist vorzugsweise nur mit einer einzigen radikalisch polymerisierbaren Gruppe substituiert. Bei Verwendung von mit zwei oder mehr radikalisch polymerisierbaren Gruppen substituierten Monomeren können vernetzte Polymerisate erhalten werden.

Erfindungsgemäße, die Monomerart (A) enthaltende Copolymere weisen vorzugsweise die allgemeine Formel (I) auf

-[CH₂-C(Y)CO-D-A]ₘ-[B]ₙ- (I)

wobei
- Y: für R, CH2COOR oder COOR und R für H oder für einen C1- bis C18-Kohlenwasserstoffrest steht,
- D: für Sauerstoff oder Stickstoff steht,
- A: für ein Saccharid, eine Saccharidderivat oder für einen Zuckeralkohol steht und eine oder mehrere Schutzgruppen enthalten kann,
- B: für ein radikalisch polymerisierbares Monomer steht, welches keine Gruppe A enthält und
- m und n: für Zahlen stehen, die den jeweiligen Monomergehalt angeben, wobei m größer Null und n größer oder gleich Null ist.
Der Rest Y ist vorzugsweise ausgewählt aus Wasserstoff, Methyl, -COOH, -COOCH₃, -COOC₂H₅, -CH₂COOCH₃ und -CH₂COOC₂H₅. Die Zahlen m und n sind vorzugsweise so gewählt, daß sich für das Gesamtpolymer ein mittleres Molekulargewicht von 10.000 bis 5.000.000 ergibt.

Monomerart (A) und Comonomer (B) liegen vorzugsweise in einem Verhältnis von 2:98 bis 98:2, besonders bevorzugt von 20:80 bis 80:20, ganz besonders bevorzugt von 30:70 bis 70:30 vor.

Die Monomerart (A) bzw. der Rest A in Formel (I) kann von einem Mono-, Di-, Oligo- oder Polysaccharid abgeleitet sein, ist aber vorzugsweise von einem Monosaccharid oder einem Zuckeralkohol oder deren Derivaten abgeleitet. Derivate sind chemisch geschützte oder enzymatisch oder chemisch modifizierte Verbindungen. Geeignete Derivate sind beispielsweise solche, die an den OH-Gruppen alkyliert, acyliert oder mit Schutzgruppen versehen sind. Besonders bevorzugt sind Monosaccharide oder Zuckeralkohole mit fünf Hydroxylgruppen, die eine solche Konfiguration aufweisen, daß zweimal zwei Hydroxylgruppen durch je eine Alkylidengruppe geschützt werden können und die fünfte Hydroxylgruppe mit der radikalisch polymerisierbaren Gruppe substituiert ist. Besonders bevorzugt sind Derivate von Glucose, Fructose, Galactose, Sorbose oder Xylit, welche eine oder mehrere, vorzugsweise zwei zweiwertige Schutzgruppen enthalten können. Bevorzugte Schutzgruppen sind Cyclohexyliden und insbesondere Isopropyliden. Es können jeweils sowohl die D- als auch die L-Enantiomeren oder ein D,L-Enantiomerengemisch eingesetzt werden.

Vorzugsweise weist die Monomerart (A) die allgemeine Formel (III) auf 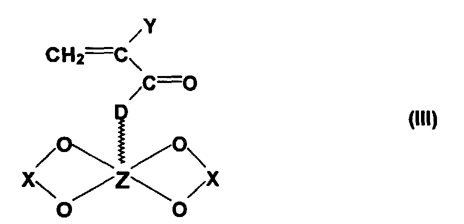 wobei Z für einen von einem Saccharid, einem Saccharidderivat oder einem Zuckeralkohol abgeleiteten Rest steht, insbesondere für ein Monosaccharid, ein Monosaccharidderivat oder einen Zuckeralkohol, vorzugsweise einen Glucose- oder Fructoserest, D für Stickstoff oder vorzugsweise für Sauerstoff steht, X eine chemische Schutzgruppe, vorzugsweise eine Isopropyliden- oder eine Cyclohexylidengruppe und Y einen der Reste R, CH2COOR oder COOR bedeuten und R Waserstoff oder einen C₁- bis C₁₈-Alkylrest bedeutet. Vorzugsweise ist Y Wasserstoff oder Methyl.

Beispiele für als Monomerart (A) geeignete Saccharidacrylate sind
- 3-O-(Meth)acryloyl-1,2:5,6-di-O-isopropyliden-α-D-glucofuranose (3-MDG bzw. 3-ADG) und seine Derivate wie z.B. 3-O-(Meth)acryloyl-1,2:5,6-di-O-cyclohexyliden-α-D-glucofuranose, wenn als Rest Z D-Glucose das Ausgangsmaterial darstellt,
- 3-O-(Meth)acryloyl-1,2:4,5-di-O-isopropyliden-α-D-fructopyranose (3-MDF bzw. 3-ADF) und seine Derivate wie z.B. 3-O-(Meth)acryloyl-1,2:4,5-di-O-cyclohexyliden-α-D-fructopyranose, wenn als Rest Z D-Fructose das Ausgangsmaterial darstellt,
- 1-O-(Meth)acryloyl-2,3:4,5-di-O-isopropyliden-α-D-fructopyranose (1-MDF bzw. 1-ADF) und seine Derivate wie z.B. 1-O-(Meth)acryloyl-2,3:4,5-di-O-cyclohexyliden-α-D-fructopyranose, wenn als Rest Z D-Fructose das Ausgangsmaterial darstellt,
- 1-O-(Meth)acryloyl-2,3:4,6-di-O-isopropyliden-α-D-sorbofuranose (1-MDS bzw. 1-ADS) und seine Derivate wie z.B. 1-O-(Meth)acryloyl-2,3:4,6-di-O-cyclohexyliden-α-D-sorbofuranose, wenn als Rest Z D-Sorbose das Ausgangsmaterial darstellt,
- 6-O-(Meth)acryloyl-1,2:3,4-di-O-isopropyliden-α-D-galactopyranose (6-MDGa bzw. 6-ADGa) und seine Derivate wie z.B. 6-O-(Meth)acryloyl-1,2:3,4-di-O-cyclohexyliden-α-D-galactopyranose, wenn als Rest Z D-Galactose das Ausgangsmaterial darstellt,
- 5-O-(Meth)acryloyl-1,2:3,4-di-O-isopropyliden-α-DL-xylit (5-MDXy bzw. 5-ADXy) und seine Derivate wie z.B. 5-O-(Meth)acryloyl-1,2:3,4-di-O-cyclohexyliden-α-DL-xylit, wenn als Rest Z ein Xylit das Ausgangsmaterial darstellt,
- 3-O-(Meth)acryloyl-1,2:4,5-di-O-isopropyliden-xylitol (3-MDXy bzw. 3-ADXy) und seine Derivate wie z.B. 3-O-(Meth)acryloyl-1,2:4,5-di-O-cyclohexyliden-xylitol, wenn als Rest Z ein Xylit das Ausgangsmaterial darstellt.

Das Comonomer B ist vorzugsweise ausgewählt aus den folgenden Gruppen, wobei auch Mischungen der genannten Monomer eingesetzt werden können:
(a) monoethylenisch ungesättigte C3- bis C12-Carbonsäuren oder deren Salze,
(b) monoethylenisch ungesättigte Ester von C3- bis C12-Carbonsäuren und C1- bis C14-Alkoholen,
(c) Acrylsäure- oder Methacrylsäuredialkylaminoalkylester mit insgesamt bis zu 30 C-Atomen im Dialkylaminoalkyl-Rest, welche in N-quaternisierter Form oder in Salzform vorliegen können,
(d) Amide der unter (a) genannten ungesättigten Carbonsäuren, z.B. Acrylsäureamid, Methacrylsäureamid, N,N-Dialkyl-acrylsäure- oder -methacrylsäureamid,
(e) fünf- bis achtgliedrige N-Vinyllactame, welche am Ring durch bis zu drei C1- bis C12-Alkylreste substituiert sein können,
(f) Maleinsäure- und Fumarsäuredialkylester mit bis zu 12 C-Atomen pro Alkylrest,
(g) monoethylenisch ungesättigte C3- bis C12-Alkylvinylether,
(h) Vinylaromaten, wie z.B. Styrol und Styrolderivate, welche am aromatischen Ring durch ein bis drei C1- bis C12-Alkylreste substituiert sein können,
(i) ein- oder mehrfach ungesättigte Kohlenwasserstoffe, wie z.B. Ethylen, Propylen oder 1,3-Butadien,
(j) Vinylhalogenide, wie z.B. Vinylchlorid.

Geeignete Comonomere (B) sind beispielweise:
(a) Acrylsäure, Methacrylsäure, Dimethylacrylsäure, Ethylacrylsäure, Itaconsäure, Vinylessigsäure, Allylessigsäure und Vinylpropionsäure, Fumarsäure, Maleinsäure. Vorzugsweise verwendet man aus dieser Gruppe Acrylsäure, Methacrylsäure, deren Gemische sowie die Natrium-, Kalium-, Calcium- oder Ammoniumsalze oder deren Mischungen.
(b) in dieser Gruppe finden sich z.B. Alkyl-, Hydroxyalkyl- und Vinylester wie Methacrylat, Ethylacrylat, n-Propylacrylat, Isopropylacrylat, n-Butylacrylat, tert.-Butylacrylat, 2-Ethylhexylacrylat, Methylmethacrylat, Ethylmethacrylat, Isopropylmethacrylat, n-Butylmethacrylat, Isobutylmethacrylat, Hydroxyethylacrylat, Hydroxypropylacrylat, Hydroxybutylacrylat, Hydroxyethylmethacrylat, 2-Ethylhexylmethacrylat und Vinylformiat, Vinylacetat, Isopropenylacetat, Vinylpropionat, Vinyl-2-ethylhexanoat, Vinyllaurat sowie Mischungen derselben Verbindung.
(c) in dieser Gruppe kommen z.B. in Betracht: Dimethylaminoethylacrylat, Diethylaminoethylacrylat, Methylethylaminoethylacrylat, Di-tert.-butylaminoethylacrylat, Dimethylamino-methyl(oder butyl, hexyl, octyl, stearyl)-acrylat, Dimethyl-(oder Diethyl, Methylethyl, Di-tert.-butyl)-aminoethylmethacrylat, Dimethylaminomethyl-(oder butyl, amyl, hexyl, octyl, stearyl)-methacrylat.
(d) Acrylsäureamid, Methacrylsäureamid, N,N-Dimethylacrylsäureamid, N,N-Dimethyl-methacrylsäureamid.
(e) Als N-Vinyllactame sind beispielsweise 1-Vinylpyrrolidon, 1-Vinylcaprolactam, 1-Vinylpiperidon, 4-Methyl-1-vinylpyrrolidon, 3,5-Dimethyl-1-vinylcaprolactam geeignet.
(f) Fumarsäurediethylester, Fumarsäuredimethylester, Fumarsäuredicyclohexylester, Maleinsäurediethylester, Maleinsäuredimethylester.
(g) in dieser Gruppe kommen z.B. in Betracht: Alkyl- und Hydroxyalkylvinylether wie Methyl-, Ethyl-, Propyl-, Isopropyl-, Butylvinylether, Hydroxyethylvinylether, Hydroxypropylvinylether, Hydroxybutylvinylether.

In einer besonderen Ausführungsform enthält das erfindungsgemäße kosmetische Mittel ein Polymerlatex, enthaltend in Wasser dispergierte Komposit-Polymerpartikel, insbesondere solche mit einer Kern/Schale-Morphologie. Diese Komposit-Polymerpartikel sind durch mindestens zweistufige Emulsionspolymerisation hergestellt und aufgebaut aus einem oder mehreren Polymeren, wobei mindestens ein Polymer in radikalisch polymerisierter Form aufgebaut ist aus mindestens einer der oben näher beschriebenen Monomerart (A). Die Komposit-Polymerpartikel weisen vorzugsweise eine Kern/Schale-Morphologie auf, aber auch von dieser Idealform abweichende Morphologien sind möglich.

Bei den erfindungsgemäßen Komposit-Polymerlatices kann das die Monomerart (A) enthaltende Polymer ein Homopolymer sein oder es kann ein durch Copolymerisation mit mindestens einem Monomer der oben näher beschriebenen Monomerart (B) erhältliches Polymer sein. Es kann sowohl im Polymerisat der ersten Stufe (Kern) als auch im Polymerisat der zweiten Stufe (Schale) enthalten sein oder es ist nur im Polymerisat der ersten Stufe (Kern) enthalten und das Polymerisat der zweiten Stufe (Schale) ist aus einem oder mehreren, beliebigen anderen Polymeren aufgebaut, welche keine Monomerart (A) enthalten. Bevorzugt sind jedoch Komposit-Polymerpartikel, bei denen das Polymerisat der ersten Stufe (Kern) aus einem oder mehreren Polymeren aufgebaut ist, welche keine Monomerart (A) enthalten und das Polymerisat der zweiten Stufe (Schale) aus mindestens einem Homo- oder Copolymer gebildet wird, welches mindestens eine Monomerart (A) enthält.

Für den Aufbau der Komposit-Polymerpartikel geeignete Homopolymere aus der Monomerart (A) weisen vorzugsweise die allgemeine Formel (II) auf

-[CH₂-C(Y)CO-D-A]ₘ- (II)

wobei
- Y: für R, CH2COOR oder COOR und R für H oder für einen C1- bis C18-Kohlenwasserstoffrest steht,
- D: für Stickstoff oder vorzugsweise Sauerstoff steht,
- A: für ein Saccharid, ein Saccharidderivat oder für einen Zuckeralkohol steht und eine oder mehrere Schutzgruppen enthalten kann und
- m: für eine Zahl steht, die den Polymerisationsgrad angibt und vorzugsweise so gewählt ist, daß sich für das Gesamtpolymer ein mittleres Molekulargewicht von 10.000 bis 5.000.000 ergibt.

Für den Substituenten A sind die gleichen Gruppen geeignet und bevorzugt wie oben bei Formel (I) beschrieben. Vorzugsweise steht Y für Wasserstoff oder Methyl, A für ein Monosaccharid, ein Monosaccharidderivat oder einen Zuckeralkohol, insbesondere für Glucose, Fructose, Galactose, Sorbose oder Xylit. Bei den Schutzgruppen, von denen vorzugsweise zwei pro Zuckermolekül vorhanden sind, handelt es sich vorzugsweise um Isopropyliden oder um Cyclohexyliden.

Der Durchmesser der Komposit-Polymerpartikel bzw. der Kern/Schale-Polymerisatteilchen beträgt vorzugsweise 40 bis 1000 nm und der Gewichtsanteil des Polymerisats der zweiten Stufe (Schale) beträgt vorzugsweise 3 bis 50%, besonders bevorzugt 10 bis 30% des Gesamtgewichts der Partikel.

Die in den erfindungsgemäßen kosmetischen Mitteln eingesetzten Saccharidpolymerlatices können wie folgt hergestellt werden. Hierbei werden die Monomere zur Bildung des dispers verteilten Polymerisats mittels Emulsionspolymerisation radikalisch polymerisiert, d.h. das Polymerisat wird aus den wenigstens eine ethylenisch ungesättigte Gruppe aufweisenden Monomeren im Beisein von Emulgatoren und gegebenenfalls weiteren, beispielsweise polymeren Dispergiermitteln sowie radikalischen Polymerisationsinitiatoren unmittelbar in wässrigem Medium in disperser Verteilung befindlich erzeugt.

Hierbei kann das Verfahren so gesteuert werden, daß Saccharidlatices mit maßgeschneiderten Eigenschaften hergestellt werden:
- Die gewünschte Endzusammensetzung der Saccharidlatices kann in Hinblick auf die gewünschten Eigenschaften und industriellen Anwendungen gezielt beim Herstellungsverfahren eingestellt werden. Beispielsweise kann durch Verwendung von verschiedenen Mengen oder verschiedenartigen hydrophoben Comonomeren wie z.B. Acrylatestern, Styrol oder Butadien die Hydrophilie/Hydrophobie der Latices eingestellt werden.
- Je nach gewünschen Eigenschaften können Saccharidlatices mit relativ niedrigem Molekulargewicht (kleiner als etwa 20.000), mittlerem Molekulargewicht (etwa 20.000 bis 100.000) oder mit hohem Molekulargewicht (über 100.000) hergestellt werden
- Durch Wahl der Monomere und Comonomere können die mit der Monomerzusammensetzung und Morphologie eng zusammenhängenden thermische Eigenschaften der erfindungsgemäßen Saccharidlatices definiert eingestellt werden.
- Die gewünschte Verträglichkeit (Löslichkeit) oder Verdünnbarkeit solcher wässrigen Dispersionen mit niedermolekularen Verbindungen wie z. B. Alkoholen, insbesondere Ethanol oder Isopropanol, bei der Formulierung kann erreicht werden, indem man einerseits verschiedene ionische und/oder nichtionische Emulgatoren oder andererseits neben den Zuckeracrylatmonomeren ein, zwei oder drei weitere Comonomere mit verschiedener Hydrophilie oder Hydrophobie einsetzt.
- Bei vielen Emulsionen sind die rheologischen Eigenschaften von großer Bedeutung. Viskosität und Streichfähigkeit sind entscheidende Qualitätsmerkmale. Diese können im wesentlichen durch die Einstellung der Partikelgröße sowie der Partikelgrößenverteilung beeinflußt werden.
- Auch weitere Qualitätsmerkmale der Emulsionen wie die optischen Eigenschaften (z.B. Farbe, Glanz), die Wirkstofffreisetzung sowie die Verteilung von Inhaltstoffen lassen sich durch geeignete Wahl der Partikelgröße gestalten oder beeinflussen.

Die Emulsionspolymerisation kann nach bekannten Verfahren zur Polymerisation in wässriger Emulsion erfolgen, wie z.B.
a) in einem Batch-Verfahren, bei dem die gesamte Monomerzugabe in einer einzelnen Charge erfolgt,
b) in einem halb-kontinuierlichen Verfahren, z.B. dem sogenannten Prä-Emulsionverfahren, bei dem zunächst nur ein Teil der Monomeren zugegeben und polymerisiert wird und die weitere Monomerzugabe inkrementeil erfolgt oder
c) in einem kontinuierlichem Verfahren, bei dem die Monomerzugabe kontinuierlich über einen längeren Zeitraum hinweg erfolgt oder
d) in einem Verfahren, welches zweistufig ist, wobei in der ersten Stufe eine Dispersion eines Saat-Polymerisats gebildet oder vorgelegt wird und in einer zweiten Stufe hieraus durch Emulsionspolymerisation ein Komposit-Polymerlatex gebildet wird oder
e) in einem Verfahren, welches als sogenanntes Shot-Growth-Verfahren ausgestaltet ist, wobei zunächst eine erste Monomerart oder ein erstes Monomergemisch teilweise polymerisiert wird und anschließend, zu einem Zeitpunkt, zu dem das erste Monomer oder die erste Monomerart noch nicht vollständig umgesetzt ist, eine zweite Monomerart oder ein zweites Monomergemisch zugegeben und copolymerisiert wird.

Die gesamte Menge des wässrigen Mediums mit den Polymerisationszusätzen kann vor Einführung der Monomeren im Polymerisationsgefäß vorliegen, oder das wässrige Medium oder ein Teil hiervon kann alternativ kontinuierlich oder inkrementell im Verlauf der Polymerisation zugegeben werden. Es können übliche Saat-Verfahren zur Unterstützung der Steuerung der Polymerisation eingesetzt werden, um die gewünschte durchschnittliche Teilchengröße und Teilchengrößenverteilung zu erreichen. Falls ein Saat-Verfahren eingesetzt wird, liegt die Polymersaat typischerweise in einer Menge vor, die etwa 0,1 bis 60 Gew.% des gesamten Polymers entspricht, und die Größe der Saatpartikel reicht typischerweise von etwa 20% bis 80% des Durchmessers der herzustellenden Polymerteilchen. Der Saatlatex kann aus einem zuvor hergestellten Latex oder aus einem dispergierten Polymerpulver bestehen, oder er kann in situ hergestellt werden. Die Monomerzusammensetzung des Saatlatex kann variieren, es ist jedoch bevorzugt, daß sie im wesentlichen der des Polymers entspricht.

Die bei der Herstellung der Latex-Polymere einzusetzenden Monomere sowie gegebenenfalls die Comonomere und wahlweise die Saat werden unter ausreichender Bewegung in Wasser dispergiert, um das Gemisch zu emulgieren. Das wässrige Medium kann auch einen radikalischen Initiator, ein Emulgiermittel (Tensid) oder andere Bestandteile enthalten, die bekannt sind und üblicherweise in der Technik als Hilfsstoffe für Emulsionspolymerisationen eingesetzt werden. Die Polymerisation wird durch Radikalbildner in einem Temperaturbereich von 0 bis 90°C initiiert, vorzugsweise unter Erwärmen des emulgierten Gemisches unter ständiger Bewegung auf eine Temperatur von üblicherweise zwischen etwa 20 und 90°C, vorzugsweise zwischen 40 und 80°C, besonders bevorzugt zwischen 50 und 80°C. Die Polymerisation wird fortgeführt, indem das emulgierte Gemisch auf der gewählten Temperatur gehalten wird, bis der gewünschte Umsetzungsgrad für die Umwandlung des Monomers oder der Monomere zum Polymer erreicht worden ist. Mach Beendigung der Monomerzugabe kann solange Initiator nachdosiert werden, bis der Restmonomergehalt bezogen auf das Gesamtgewicht des Latex unter 1 Gew.% liegt. Vorzugsweise wird die Polymerisation des Latex so geführt, daß der Restmonomergehalt unter 1 Gew.%, bezogen auf das Gesamtgewicht des Latex beträgt und ein Feststoffgehalt von ca. 20 bis 65 Gew.% resultiert.

Bezogen auf den bei der Polymerisation eingesetzten Gesamtgehalt an Monomeren verwendet man vorzugsweise 0,01 bis 20, besonders bevorzugt 0,1 bis 10 Mol.-% eines geeigneten radikalischen Polymerisationsinitiators oder einer Mischung mehrerer Polymerisationsinitiatoren. Man kann wasserlösliche sowie wasserunlösliche Initiatoren oder Mischungen von wasserlöslichen und wasserunlöslichen Initiatoren einsetzen. Die in Wasser unlöslichen Initiatoren sind dann in der dispergierten organischen Phase (Ölphase) löslich. Als Initiatoren kommen zur Herstellung der erfindungsgemäßen Polymerdispersion prinzipiell alle diejenigen in Betracht, die in der Lage sind, eine radikalische, wässrige Emulsionspolymerisation auszulösen. Es kann sich dabei sowohl um Peroxide als auch um Persulfate, Peroxodisulfate oder um Azoverbindungen handeln. Besonders geeignete Peroxide sind Wasserstoffperoxid, tert.-Butylperoxid, Diisopropylbenzolhydroperoxid, Para-Menthanhydroperoxid, Cumolhydroperoxid und Peroxodischwefelsäure sowie deren Salze. Geeignete Azoverbindugnen sind Azobisisobutyronitril oder Azobiscyanovaleriansäure.

Falls man die Polymerisation zunächst bei niedriger Temperatur startet und bei höherer Temperatur zu Ende führt, ist es zweckmäßig, mit mindestens zwei bei verschiedenen Temperaturen zerfallenden Initiatoren zu arbeiten, nämlich zunächst mit einem bei niedriger Temperatur zerfallenden Initiator zu beginnen und dann die Hauptpolymerisation mit einem Initiator zu Ende zu führen, der bei höherer Temperatur zerfällt. Für die folgenden angegebenen Temperaturbereiche kann man beispielsweise die dafür aufgeführten Initiatoren verwenden:

### Temperatur 40 bis 60°C:

Acetylcyclohexansulfonylperoxid, Diacetyl-, Dicyclohexyl- oder Di-2-ethylhexylperoxidicarbonat, tert.-Butyl- oder tert.-Amylperneodecanoat, 2,2'-Azobis-(4-methoxy-2,4-dimethyl-valeronitril), 2,2'-Azobis-(2-amidinopropan)-dihydrochlorid, 2,2'-Azobis-(2-(2-imidazolin-2-yl)-propan)-dihydrochlorid;

### Temperatur 60 bis 80°C:

Natriumpersulfat, Kaliumpersulfat, Ammoniumpersulfat, Tert.-Butyl-, tert.-Amylperpivalat, Dioctanoyl- oder Dilaurylperoxid, 2,2'-Azobis-(2,4-dimethylvaleronitril), 2,2'-Azobis-(isobutyronitril);

### Temperatur 80 bis 90°C:

Dibenzoylperoxid, tert.-Butylper-2-ethylhexanoat, tert.-Butylpermaleinat, Dimethyl-2,2'-azobis-isobutyrat.

Wenn man die Halbwertszeiten der angegebenen radikalbildenden Initiatoren verringern will, verwendet man zusätzlich zu den genannten Initiatoren noch als Redoxkatalysatoren geeignete Salze, d.h. kombinierte Systeme, die aus wenigstens einem Reduktionsmittel und wenigstens einem Oxidationsmittel zusammengesetzt sind. Geeignete Oxidationsmittel sind beispielsweise Peroxide oder Hydroperoxide wie z.B. Benzoylperoxid oder Wasserstoffperoxid, Kalium-, Natrium- oder Ammoniumpersulfat. Die Reduktionsmittel aktivieren die Radikalbildung und ermöglichen so die Durchführung der Emulsionspolymerisation bei tieferen Temperaturen, beispielsweise bei 30 bis 55°C. Als Redoxkomponente setzt man bezogen auf den Initiator 0,01 bis 50 Mol.-% der reduzierend wirkenden Verbindungen ein. Die reduzierende Komponente von Redoxkatalysatoren kann beispielsweise von Verbindungen wie Natriumsulfit, Natriumbisulfit, Alkalithiosulfat, Natriumformaldehydsulfoxylat oder Hydrazin gebildet werden. Weitere geeignete Reduktionsmittel sind Ascorbinsäure, Acetonbisulfit, das Natriumsalz der Hydroxymethansulfinsäure oder Natriumdithionit. Bevorzugt sind auch kombinierte Systeme mit einem zusätzlichem Gehalt an einer geringen Menge einer löslichen Metallverbindung, welche in mehreren Wertigkeitsstufen auftreten kann, beispielsweise Eisen(II)salze wie Eisen(II)sulfat. Anstelle eines wasserlöslichen Eisen(II)salzes wird häufig auch eine Kombination von Fe/V-Salzen benutzt. Der Vorteil dieser Systeme liegt darin, daß sie eine Durchführung der Emulsionpolymerisation bei noch niedrigeren Temperaturen ermöglichen. Beispiele für derartige Systeme sind Ascorbinsäure/Eisen(II)sulfat/Wasserstoffperoxid oder Natriumdithionit/Natriumformaldehydsulfoxylat/Eisen(II)sulfat/ Para-Menthanhydroperoxid oder Diisopropylbenzolhydroperoxid. Häufig wird derartigen Systemen noch ein Chelatbildner zugesetzt, um sicherzustellen, daß sich die metalische Komponente in Lösung befindet und dem Reaktionssystem nicht durch Ausfällung entzogen wird. Ein solcher Chelatbildner ist z.B. das Natriumsalz der Ethylendiamintetraessigsäure. Häufig wird die metallische Komponente unmitelbar als Chelatkomplex zugesetzt.

Zur Erhöhung der Stabilität der Polymerdispersion, insbesondere bei höheren Feststoffgehalten, können sowohl die zur Durchführung von radikalischen, wässrigen Emulsionspolymerisationen üblicherweise eingesetzten Schutzkolloide als auch Emulgatoren oder deren Mischungen eingesetzt werden. Die vorzugsweise wasserlöslichen Stabilisatoren können bezogen auf den Monomergehalt in Mengen von 1 bis 20 Gew.% zugesetzt werden. Geeignete Schutzkolloide sind beispielsweise Polyvinylalkohole, Cellulosederivate, Vinylpyrrolidon enthaltende Copolymere oder Polykondensate aus Naphtalinsulfonsäure und Formaldehyd mit einem mittleren relativen Molekulargewicht von 4000 bis 8000.

Als geeignete Emulgatoren, die im Unterschied zu den Schutzkolloiden als grenzflächenaktive Substanzen Micellen bilden können, werden bevorzugt solche eingesetzt, deren Molekulargewichte im Gegensatz zu den Schutzkolloiden üblicherweise unter 2000, vorzugsweise unter 1000 liegen. Die Tenside werden in einer Menge von 1 bis 20 Gew.% eingesetzt. Die Emulgatoren können nichtionisch, anionisch oder kationisch sein. Mischungen können eingesetzt werden, soweit sie miteinander verträglich sind, was in der regel der Fall ist mit Ausnahme von Mischungen von kationischen und anionischen Emulgatoren, die häufig miteinander unverträglich sind. Üblicherweise wird mindestens ein anionischer Emulgator verwendet. Ein oder mehrere nichtionische Emulgatoren können zusätzlich verwendet werden. Geeignete anionische Emulgatoren sind z.B. Alkali- und Ammoniumsalze von Alkylsulfaten, insbesondere von C8- bis C16-Alkylsulfaten, von Aryl- und Alkylarylsulfonaten, insbesondere C9- bis C18-Alkylarylsulfonaten, von Schwefelsäurehalbestern ethoxylierter Alkanole und Alkylphenole, insbesondere von C12- bis C18-Alkanolen mit einem Ethoxylierungsgrad von 1 bis 70, von C12- bis C18-Alkylsulfonsäuren, von Arylsulfonsäuren, von C9- bis C18-Alkylarylsulfonsäuren und von C10- bis C18-Alkylsulfaten, sowie sulfonierte Alkylester und Fettsäureseifen. Geeignete nichtionische Emulgatoren sind z.B. ethoxylierte Mono-, Di- und Tri-C4- bis C12-alkylphenole mit einem Ethoxylierungsgrad von 3 bis 100 oder ethoxylierte C8- bis C18-Fettalkohole mit eidnem Ethoxylierungsgrad von 3 bis 100. Weiterhin sind Sulfobernsteinsäureester geeignet. Spezielle Beispiele geeigenter Emulgatoren sind u.a. Natriumlaurylsulfat, Natriumdodecylbenzolsulfonat, Natriumbutylnaphthalinsulfonat, Dinatriumdodecyldiphenylethersulfonat, N-Octadecyldinatriumsulfosuccinat und Dioctylnatriumsulfosuccinat. Die Emulgiermittel werden in geeigneten Mengen eingesetzt, um eine angemessene Emulgierung zu erreichen und die gewünschte Teilchengröße und Teilchengrößenverteilung zu liefern.

Weiterhin können bezogen auf den Monomergehalt 0,1 bis 10 Gew.%, vorzugsweise 0,5 bis 5 Gew.% an multifunktionellen Verbindungen, insbesondere mindestens zweifach ethylenisch ungesättigten, quervernetzenden Verbindungen oder Pfropfungsmitteln zugesetzt werden. Geeignete Pfropfungsmittel sind beispielsweise Acryl- oder Methacrylsäureallylester, Acryl- oder Methacrylsäuremethallylester, Fumar-, Malein- oder Itaconsäuremono- oder -diallylester oder Fumar-, Malein- oder Itaconsäuremono- oder -dimethallylester.

Zur Begrenzung des Vernetzungs- und/oder Polymerisatonsgrades können der zu polymerisierenden Mischung bezogen auf den Monomergehalt 0,01 bis 10, bevorzugt 0,1 bis 3 Mol% Regler, sogenannte Molekulargewichtsregler (Kettenüberträger) zugesetzt werden. Geeignete Regler sind Mercaptane, insbesondere C3- bis C15-Alkanthiole, sowie Aldehyde und Chlorkohlenwasserstoffe. Bevorzugt sind tert.-Docedylmercaptan und n-Dodecylmercaptan.

Die Emulsionspolymerisation erfolgt üblicherweise in einer Inertgasatmosphäre unter Ausschluß von Luftsauerstoff. Das Wasser wird zweckmäßig unmittelbar vor dem Polymerisationsansatz frisch destilliert und entgast. Die Monomerarten (A) und (B) sind vorzugsweise rein und stabilisatorfrei und liegen in gelöster Form vor. Während der Polymerisation wird für eine gute Durchmischung der Reaktionsteilnehmer gesorgt. Die Herstellung der erfindungsgemäßen Saccharidlatices kann in üblichen Polymerisationsvorrichtungen durchgeführt werden. Hierzu verwendet man beispielsweise Rührkessel oder Doppelwandreaktoren, die mit einem Anker-, Blatt-, Impeller- oder einem mehrstufigen Impulsgegenstromrührer ausgestattet sind.

Die radikalische, wässrige Emulsionspolymerisation zur Erzeugung der erfindungsgemäß einzusetzenden Polymerlatices wird vorteilhafterweise so durchgeführt, daß man die Gesamtmenge des Polymerisationsansatzes (einschließlich gegebenenfalls des Molekulargewichtsreglers) abzüglich des Radikalbildners in das Polymerisationsgefäß vorlegt, auf die Polymerisationstemperatur erwärmt und anschließend (in der Regel auf einmal) Radikalbildner in das Polymerisationsgefäß einbringt und bis zu dem gewünschten Endumsatz, beispielweise 90% oder 98% polymerisiert.Anschließend kann die Emulsionpolymerisation durch Zusatz von Polymerisationsinhibitoren wie Diethylhydroxylamin gestoppt und nicht umgesetztes Monomer in bekannter Weise durch Desodorieren (vorzugsweise Strippen und/oder Wasserdampfdestillation) entfernt werden. Während der Polymerisation kann auch zur Erreichung des gewünschten Endumsatzes und zur zusätzlichen Stabilisation der Polymerdispersion weiterer Radikalbildner und/oder Dispergiermittel zugesetzt werden. Zur Stabilisierung des pH-Wertes können während der Polymerisation Puffer wie z.B. Alkaliphosphat zugesetzt werden. Ein Zusatz geringer Mengen starker Elektrolyte wie Kaliumsulfat, Kaliumchlorid und/oder Natriumsulfat erleichert in an sich bekannter Weise die Einstellung der gewünschten Teilchendurchmesser des Polymerisats. Im übrigen wird der Teilchendurchmesser des Polymerisats hauptsächlich durch die Menge des verwendeten Dispergiermittels bestimmt. In der Regel gehen mit zunehmender Dispergiermittelmenge abnehmende Teilchendurchmesser einher.

Im Anschluß an die Polymerisation kann der Feststoffgehalt des erhaltenen wässrigen heterogenen Polymerlatex durch Zugabe von Wasser oder Entzug von Wasser durch Destillation auf das gewünschte Niveau eingestellt werden. Die wässrigen Dispersionen können mit protischen Lösungsmitteln verdünnt werden. Als polare protische Lösemittel eignen sich beispielsweise Methanol, Ethanol, Isopropanol, Ethylenglycol, Propylenglycol, Glycerin, 1,3-Butylenglycol, 2,3-Butylenglycol, sowie Mischungen der genannten Alkohole. Im allgemeinen liegt das gewünschte Niveau des Polymerfeststoffgehalts bei etwa 20 bis 60 Gew-%, bezogen auf das Gesamtgewicht. Da die Emulsionspolymerisation ausschließlich in Wasser durchgeführt wird, ist diese Technik auf einen großen Maßstab übertragbar. In diesem Fall liegen die Monomerarten (A) und (B) und die gebildeten Polymerlatexpartikel im Wasser in sphärischer Form vor.

Die Herstellung der erfindungsgemäß einzusetzenden Komposit-Polymerlatices erfolgt durch bekannte, zweistufige Emulsionspolymerisation. Hierbei wird in einer ersten Stufe mit Hilfe der Emulsionspolymerisation eine Polymerdispersion erzeugt oder es wird ein bereits fertiges, beispielsweise im Handel erhältliches, unlösliches Polymer als Saatlatex in einem wässrigen Medium dispergiert. In der zweiten Stufe wird durch Zudosieren der üblichen Initiatoren sowie weiterer, radikalisch polymerisierbarer Monomere eine Hülle eines weiteren Polymeren in wässriger Emulsion aufgepfropft. Dabei ist mindestens eines der das Polymerisat der ersten Stufe (d.h. den Kern von Kern/Schale-Polymerpartikeln) oder das Polymerisat der zweiten Stufe (d.h. die Schale von Kern/Schale-Polymerpartikeln) bildenden Polymere aus mindestens einer Monomerart (A) aufgebaut ist, welche eine mit mindestens einer radikalisch polymerisierbaren Gruppe substituierte Verbindung ist, welche ausgewählt ist aus den oben näher erläuterten Kohlenhydraten oder Kohlenhydratderivaten, wobei die Monomerart eine oder mehrere Schutzgruppen enthalten kann. Die Menge an den Kern bildenden Polymeren und die Menge an die Schale bildenden Monomeren werden typischerweise so gewählt, daß die Schale 3 bis 95 Gew.%, vorzugsweise 5 bis 50 Gew.%, besonders bevorzugt 10 bis 30 Gew.% der Gesamtmenge der Kern/ Schale-Partikel ausmacht.

Vorzugsweise wird die Schale aus einem Homopolymer gemäß der oben angegebenen allgemeinen Formel (II) oder aus einem mit der Monomerart (B) copolymeriserten Polymer gemäß der oben angegebenen allgemeinen Formel (I) gebildet. Hierfür werden als ethylenisch ungesättigte Saccharidderivate der Monomerart (A) Verbindungen der oben angegebenen allgemeinen Formel (III) verwendet. Als Comonomere können die oben für die Monomerart (B) angegebenen ethylenisch ungesättigten Monomere eingesetzt werden.

Die zweistufige Emulsionspolymerisation zur Bildung der Komposit-Polymerlatices kann durch wasserlösliche oder durch öllösliche Starter gestartet werden. Bevorzugt sind öllösliche Starter, ausgewählt aus organischen Peroxiden, organischen Hydroperoxiden oder organischen Perestern. Geeignet sind beispielsweise Cumolhydroperoxid, Dicumolperoxid, tert.-Butylperneodekanoat, tert.-Butylperbenzoat oder Dioctanoyl- oder Dilauroylperoxid. Die Polymerisation der Schale erfolgt in Abhängigkeit des gewählten Initiatorsystems im Temperaturbereich von 30 bis 90°C. Gegebenenfalls können noch die oben angegebenen Emulgatoren zugegeben werden. Nach Beendigung der Monomerzugabe kann solange Initiator nachdosiert werden, bis der Restmonomergehalt bezogen auf das Gesamtgewicht des Latex unter 1 Gew.% liegt. Das Verfahrensprodukt kann direkt in Form des Latex eingesetzt und verwendet werden oder die Polymerisationsprodukte können beispielsweise durch Sprühtrocknung, Walzentrocknung oder durch Koagulation mit anschließender Trocknung aufgearbeitet und isoliert werden.

In einer bevorzugten Ausführungsform wird die zweistufige Emulsionspolymerisation zur Bildung der Komposit-Polymerlatices unter Verwendung von Pfropfungsmitteln durchgeführt, wobei die Pfropfungsmittel im Kern, in der Schale oder vorzugsweise in beiden enthalten sind. Geeignete Pfropfungsmittel sind beispielsweise Acryl- oder Methacrylsäureallylester, Acryl- oder Methacrylsäuremethallylester, Fumar-, Malein- oder Itaconsäuremono- oder -diallylester oder Fumar-, Malein- oder Itaconsäuremono- oder -dimethallylester.

Die erfindungsgemäß bevorzugt in den kosmetischen Mitteln eingesetzten Saccharidlatices weisen eine Reihe von Vorteilen gegenüber Lösungen von rein synthetischen Polymeren und von modifizierten natürlichen Polymeren auf, wie hoher Feststoffgehalt bei dennoch niedriger Viskosität, geringer Geruch, verbesserte Umweltverträglichkeit, Lösungsmittelfreiheit der wässrigen Dispersionen. Da die Latices selber bereits einen gewissen Anteil an Tensiden enthalten, kann in wässrigen kosmetischen Mitteln vorteilhafterweise auf einen sonst üblichen zusätzlichen Tensidgehalt zur Solubilisierung lipophiler Wirkstoffe ganz oder teilweise verzichtet werden.

Vorzugsweise handelt es sich bei den in dem kosmetischen Mittel enthaltenen Saccharidpolymeren um Homopolymere gemäß der oben angegebenen allgemeinen Formel (II) oder um Copolymere gemäß der oben angegebenen allgemeinen Formel (I). Diese werden gebildet aus ethylenisch ungesättigten Saccharidderivaten der Monomerart (A), insbesondere Verbindungen der oben angegebenen allgemeinen Formel (III). Als Comonomere können die oben für die Monomerart (B) angegebenen ethylenisch ungesättigten Monomere eingesetzt werden. Vorzugsweise werden die hydrophilen, von Sacchariden abgeleiteten Monomere mit hydrophoben Co-Monomeren kombiniert. Durch geeignete Wahl von Art und Mengen der Comonomere können die Löslichkeits-, Filmbildungs-, Haarfestigungs- und Haarpflegeeigenschaften sowie die Substantivität zu Haut und Haaren der resultierenden Copolymere gezielt eingestellt werden.

Für die in den erfindungsgemäßen Mitteln einzusetzenden Saccharidpolymere besonders geeignete nichtionische Comonomere sind beispielsweise Acrylamid, Methacrylamid, Alkyl- und Dialkylacrylamid, Alkyl- und Dialkylmethacrylamid, ethylenisch ungesättigte Ester von C3- bis C10-Carbonsäuren (z.B. Alkylacrylat oder Alkylmethacrylat), Maleinsäuredialkylester, Vinylcaprolacton, fünf- bis achtgliedrige N-Vinyllactame, welche am Ring durch bis zu 3 C1- bis C12-Alkylreste substituiert sein können, insbesondere Vinylpyrrolidon und Vinylcaprolactam, weiterhin Vinylalkohol und Vinylester (z.B. Vinylacetat oder Vinylpropionat), Styrol, welches am aromatischen Ring durch ein oder zwei C1- bis C3-Alkylreste substituiert sein kann. Die Alkylgruppen dieser Monomere sind vorzugsweise C1- bis C7-Alkylgruppen, besonders bevorzugt C1- bis C3-Alkylgruppen. Weitere geeignete nichtionische copolymerisierbare Einheiten sind Silikonmakromere mit einer endständigen ethylenisch ungesättigten Gruppe, wie sie beispielsweise in der EP 0 412 704 beschrieben werden. Die ungesättigten Silikonmacromere besitzen die allgemeine Formel X-(Y)ₙ-Si(R)₃₋ₘ(Z)ₘ, wobei X eine Vinylgruppe, Y eine divalente Verbindungsgruppe, R Wasserstoff, Alkyl, Aryl oder Alkoxy und Z eine monovalente Polysiloxangruppe mit einem Molekulargewicht von mindestens 500 ist und n für 0 oder 1 und m für 1 bis 3 steht. Die genannten Alkylgruppen enthalten vorzugsweise 1 bis 24, besonders bevorzugt 1 bis 6 Kohlenstoffatome.

Für die in den erfindungsgemäßen Mitteln einzusetzenden Saccharidpolymere besonders geeignete anionische Comonomere sind beispielsweise monoethylenisch ungesättigte C3- bis C10-Carbonsäuren und deren Alkalimetall-, Erdalkalimetall- oder Ammoniumsalze wie z.B. Acrylsäure, Methacrylsäure, Dimethylacrylsäure, Ethylacrylsäure, Vinylessigsäure, Allylessigsäure, Vinylpropionsäure, Crotonsäure, Maleinsäure bzw. Maleinsäureanhydrid oder Maleinsäuremonoester.

Für die in den erfindungsgemäßen Mitteln einzusetzenden Saccharidpolymere besonders geeignete kationische Comonomere sind beispielsweise Monomere, welche kationische oder kationisierbare Gruppen, vorzugsweise primäre, sekundäre, tertiäre oder quaternäre Stickstoffgruppen enthalten. Geeignete ammoniumsubstituierte Vinylmonomere sind zum Beispiel Trialkylmethacryloxyalkylammonium, Trialkylacryloxyalkylammonium, Dialkyldiallylammonium, quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, welches am heterocyclischen Ring mit bis zu 3 C1- bis C12-Alkylresten substituiert sein kann, Alkylvinylpyridinium, oder Alkylvinylpyrrolidon Salze. Geeignete aminsubstituierte Vinylmonomere sind zum Beispiel Dialkylaminoalkylacrylat, Dialkylaminoalkylmethacrylat, Monoalkylaminoalkylacrylat und Monoalkylaminoalkylmethacrylat, N-Vinylimidazol, welches am Ring mit bis zu 3 C1- bis C12-Alkylresten substituiert sein kann. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Für die in den erfindungsgemäßen Mitteln einzusetzenden Saccharidpolymere besonders geeignete zwitterionische oder amphotere Comonomere enthalten sowohl basische oder kationische Gruppen als auch saure oder anionische Gruppen. Geeignete Monomere mit zwitterionischen Gruppen enthalten die funktionelle Gruppe -NR1R2⁺-A-COO⁻, wobei R1 und R2 unabhängig voneinander Alkylgruppen mit 1 bis 24, vorzugsweise 1 bis 4 C-Atomen und A eine divalente Verbindungsgruppe, beispielsweise eine Alkylengruppe mit 1 bis 24, vorzugsweise 1 bis 4 C-Atomen bedeuten, wobei die Reste R1 und R2 so verbunden sein können, daß sie einen das N-Atom enthaltenden Ring bilden, welcher vorzugsweise 6-gliedrig ist. Ein bevorzugtes Monomer ist Methacryloylethylbetain.

Die erfindungsgemäß einzusetzenden Polymere können weiterhin über freie Hydroxylgruppen mit Polyethylenglykol- oder Polypropylenglykolketten verknüpft sein.

Die erfindungsgemäß einzusetzenden Polymere und Polymerlatices zeichnen sich durch eine hohe Affinität zu keratinischem Material, insbesondere zu Haut, Haaren und Nägeln aus. Sie legen sich beispielsweise an das Haar an und verstärken dadurch den Halt der Frisur.

Setzt man die Saccharidpolymere bzw. Polymerlatices in typischen Haarpflegemitteln ein wie Lotionen, Schäumen, Flüssig- oder Schaumfestigern, so erhält damit behandeltes Haar im trockenen Zustand gute Festigung und Spannkraft bei gleichzeitig verbessertem Volumen.

In einer bevorzugten Ausführungsform enthält das erfindungsgemäße kosmetische Mittel zusätzlich mindestens ein weiteres, von den Saccharidpolymeren verschiedenes, filmbildendes Polymer in einer Menge von vorzugsweise 0,01 bis 25 Gew.%.

Die zusätzlichen filmbildenden Polymere liegen vorzugsweise in einer Menge von 0,01 bis 25, besonders bevorzugt von 0,1 bis 20 Gewichtsprozent vor und können einzeln oder in einem Gemisch eingesetzt werden. Unter filmbildenden Polymeren sollen solche Polymere verstanden werden, die in der Lage sind, auf Haaren einen Polymerfilm abzuscheiden. Die filmbildenden Polymere können nichtionisch, kationisch, anionisch, zwitterionisch oder amphoter sein und synthetischen oder natürlichen Ursprungs sein.

Geeignete anionische Polymere sind synthetische Homo- oder Copolymere mit neutralisierbare Säuregruppen enthaltenden Monomereinheiten, welche gegebenenfalls mit Comonomeren, die keine Säuregruppen enthalten, copolymerisiert sind. Als Säuregruppen kommen Sulfonsäure-, Phosphorsäure- und Carbonsäuregruppen in Betracht, von denen die Carbonsäuregruppen bevorzugt sind. Geeignete Säuregruppen enthaltende Monomere sind beispielsweise Acrylsäure, Methacrylsäure, Crotonsäure, Maleinsäure bzw. Maleinsäureanhydrid, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete anionische Polymere sind insbesondere unvernetzte oder mit polyfunktionellen Agenzien vernetzte Homopolymere der Acrylsäure oder der Methacrylsäure, Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes anionisches, natürliches Polymer ist beispielsweise teilweise oder vollständig neutralisierter Schellack.

Bevorzugte Polymere mit Säuregruppen sind vernetzte oder unvernetzte Vinylacetat/Crotonsäure Copolymere, die beispielsweise in Form einer 60%igen Lösung in Isopropanol/Wasser unter der Handelsbezeichnung ARISTOFLEX® von der Firma HOECHST/Deutschland beziehungsweise von der Firma BASF unter dem Handelsnamen LUVISET® CA-66 vertrieben werden. Weitere geeignete anionische Polymere sind zum Beispiel Terpolymere aus Vinylacetat, Crotonsäure und Polyethylenoxid sowie Terpolymere aus Acrylsäure, Alkylacrylat und N-Alkylacrylamid, insbesondere Acrylsäure/Ethylacrylat/N-t-Butylacrylamid Terpolymere, wie sie unter den Handelsnamen ULTRAHOLD® 8 und ULTRAHOLD® STRONG der Firma BASF/Deutschland vertrieben werden oder Terpolymere aus Vinylacetat, Crotonat und Vinylalkanoat, insbesondere Vinylacetat/Crotonat/Vinylneodecanoat Copolymere, wie sie z.B. von der Firma National Starch unter der Handeisbezeichnung RESYN 28-2930 vertrieben werden.

Eine weitere Klasse von geeigneten anionischen Polymeren sind anionische Polyurethane. Bevorzugte Polyurethane sind dadurch gekennzeichnet, daß sie (a) endständige Säuregruppen besitzen, die beispielweise über Aminosulfonsäuren oder Aminocarbonsäuren eingeführt wurden, (b) gegebenenfalls weitere freie Carbonsäuregruppen enthalten, die durch Einpolymerisieren von Carbonsäurediolen wie beispielsweise Dimethylolpropansäure als Comonomere eingeführt wurden und (c) Polyurethansequenzen enthalten, die aus Polyesterdiolen und Diisocyanaten wie beispielsweise Alkylendiisocyanaten oder Isophorondiisocyanat gebildet wurden. Geeignet ist beispielsweise Luviset® PUR der Firma BASF/Deutschland.

Die anionischen Polymere liegen im erfindungsgemässen Mittel teilweise oder vollständig mit einem kosmetisch verträglichen Neutralisationsmittel neutralisiert vor. Als Neutralisationsmittel können organische oder anorganische Basen verwendet werden. Beispiele für Basen sind insbesondere Aminoalkanole wie z.B. Aminomethylpropanol (AMP), Triethanolamin oder Monoethanolamin, aber auch Ammoniak, NaOH u.a..

Geeignete synthetische, nichtionische, filmbildende, haarfestigende Polymere sind zum Beispiel Homo- oder Copolymere, welche aus mindestens einem nichtionischen Monomer aufgebaut sind. Nichtionische Monomere sind z.B. Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, wobei die Alkylgruppen dieser Monomere vorzugsweise C1- bis C7-Alkylgruppen, besonders bevorzugt C1-bis C3-Alkylgruppen sind. Geeignete synthetische, nichtionische, filmbildende, haarfestigende Polymere sind zum Beispiel Homopolymere des Vinylpyrrolidons sowie Homopolymere des N-Vinylformamids. Weitere geeignete synthetische filmbildende, nichtionische haarfestigende Polymere sind zum Beispiel Copolymerisate aus Vinylpyrrolidon und Vinylacetat, Terpolymere aus Vinylpyrrolidon, Vinylacetat und Vinylpropionat, Polyacrylamide, Polyvinylalkohole oder Polyethylenglykole mit einem Molekulargewicht von 800 bis 20.000 g/mol.

Geeignete natürliche filmbildende Polymere sind zum Beispiel Chitosan mit einem Molekulargewicht von 20.000 bis ca. 5 Millionen g/mol, wie es beispielsweise von der Firma Pronova vertrieben wird, oder verschiedene Saccharidtypen wie zum Beispiel Polysaccharide oder Gemische aus Oligo-, Mono- und Disacchariden, welche beispielsweise unter dem Handelsnamen C-PUR® von der Firma Cerestar, Brüssel/Belgien vertrieben werden. Weitere geeignete, natürliche Polymere sind chinesiches Balsamharz und Cellulosederivate, zum Beispiel Hydroxypropylcellulose mit einem Molekulargewicht von 30.000 bis 50.000 g/mol.

Geeignete filmbildende kationische Polymere sind dadurch gekennzeichnet, daß sie aus mindestens einer Monomerart aufgebaut sind, die kationische oder kationisierbare Gruppen, vorzugsweise primäre, sekundäre, tertiäre oder quaternäre Stickstoffgruppen enthält. Geeignete ammoniumsubstituierte Vinylmonomere sind zum Beispiel Trialkylmethacryloxyalkylammonium, Trialkylacryloxyalkylammonium, Dialkyldiallylammonium, quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, welches am heterocyclischen Ring mit bis zu 3 C1- bis C12-Alkylresten substituiert sein kann, Alkylvinylpyridinium, oder Alkylvinylpyrrolidon Salze. Geeignete aminsubstituierte Vinylmonomere sind zum Beispiel Dialkylaminoalkylacrylat, Dialkylaminoalkylmethacrylat, Monoalkylaminoalkylacrylat und Monoalkylaminoalkylmethacrylat, N-Vinylimidazol, welches am Ring mit bis zu 3 C1- bis C12-Alkylresten substituiert sein kann. Die Alkylgruppen dieser Monomere sind vorzugsweise niedere Alkylgruppen wie zum Beispiel C1- bis C7-Alkylgruppen, besonders bevorzugt C1- bis C3-Alkylgruppen. Die kationischen bzw. basischen Monomere können mit nicht-kationischen bzw. nicht-basischen Comonomeren copolymerisiert sein.

Geeignete kationische Polymere sind zum Beispiel Polyvinylpyrrolidon/Dimethylaminoethylmethacrylat Copolymer, ein Copolymer aus Polyvinylpyrolidon und Imidazoliminmethochlorid, ein Terpolymer aus Dimethyldiallylammoniumchlorid, Natriumacrylat und Acrylamid, ein Terpolymer aus Vinylpyrrolidon, Dimethylaminoethylmethacrylat und Vinylcaprolactam, mit quaternierten Ammoniumgruppen substituierte Hydroxyethylcellulose, Vinylpyrrolidon/Methacrylamidopropyltrimethylammoniumchlorid Copolymer oder diquaternäre Polydimethylsiloxane (INCI: Quaternium-80).

Geeignete amphotere Polymere sind Polymere, welche sowohl kationische oder durch Protonierung kationisierbare Gruppen als auch anionische oder durch Deprotonierung anionisierbare Gruppen enthalten. Kationische Gruppen sind beispielsweise quaternäre Amingruppen, kationisierbare Gruppen sind beispielsweise primäre, sekundäre oder tertiäre Amingruppen. Anionische Gruppen sind beispielsweise Carboxylat-, Sulfat-, Sulfonat-, Phosphat- oder Phosphonatgruppen. Anionisierbare Gruppen sind beispielsweise die protonierten Formen der genannten anionischen Gruppen.

Geeignete amphotere Polymere sind z.B. Copolymere aus Octylacrylamid, t-Butylaminoethylmethacrylat und zwei oder mehr Monomeren, bestehend aus Acrylsäure, Methacrylsäure oder deren Estern. Weitere Beispiele sind Copolymere von Acrylsäure, Methylacrylat und Methacrylamidopropyltrimethylammoniumchlorid (INCI: Polyquaternium-47), Copolymere aus Acrylamidopropyltrimoniumchlorid und Acrylaten oder Copolymere aus Acrylamid, Acrylamidopropyltrimoniumchlorid, 2-Amidopropylacrylamidsulfonat und DMAPA (INCI: Polyquaternium-43).

Das erfindungsgemäße kosmetische Mittel kann darüber hinaus weitere, für kosmetische Mittel übliche Zusatzbestandteile enthalten, zum Beispiel kationische Kämmbarkeitsverbesserer, Öle, Silikonöle, Verdicker und anderes mehr. Ebenso können Konsistenzgeber, wie sie üblicherweise für Cremes eingesetzt werden, enthalten sein, beispielsweise Fettalkohole, Fettalkoholsulfate oder Fettalkoholethersulfate. Ebenfalls enthalten sein können bei Raumtemperatur flüssige, wachsartige oder feste Polyethylenglykole. Selbstverständlich kann das erfindungsgemäße Mittel auch weitere übliche kosmetische Zusätze, wie verdickende Polymere sowie deren Kombination in einer Menge von vorzugsweise 0,01 bis 15 Gewichtsprozent; Parfümöle in einer Menge von vorzugsweise 0,01 bis 5 Gewichtsprozent; Trübungsmittel wie z.B. Ethylenglykoldistearat, Styrol/PVP Copolymere oder Polystyrole in einer Menge von vorzugsweise 0,01 bis 5 Gewichtsprozent; Netzmittel, Tenside oder Emulgatoren mit oder ohne Waschaktivität aus den Klassen der nichtionischen, anionischen, kationischen oder amphoteren oberflächenaktiven Substanzen wie Fettalkoholsulfate, ethoxylierte Fettalkohole, Fettsäurealkanolamide wie zum Beispiel die Ester der hydrierten Rizinusölfettsäuren in einer Menge von vorzugsweise 0,1 bis 20 Gewichtsprozent; ferner Feuchthaltemittel, Farbstoffe, Lichtschutzmittel, Antioxidantien, Glanzgeber und Konservierungsstoffe in einer Menge von vorzugsweise 0,01 - 10 Gewichtsprozent enthalten. Als weitere Zusätze sind geeignete wasserlösliche oder wasserunlösliche Silikonpolymere in einer Konzentration von 0,01 bis 50 Gewichtsprozent, bevorzugt in einer Konzentration von 0,1 bis 5 Gewichtsprozent einsetzbar.

In einer Ausführungsform liegt das erfindungsgemäße kosmetische Mittel als Haarbehandlungsmittel vor. Das erfindungsgemäße Haarbehandlungsmittel kann in verschiedenen Applikationsformen Anwendung finden, z.B. in Form von Haarpflege-, Haarfestigungs-, Haarreinigungs-, Haarfärbe- oder Tönungsmittel, als Blondiermittel oder als Dauerwellmittel. Das Mittel kann als Lotion, Schaum, Milch, Gel, Creme, Gelschaum, Aerosol- oder Nonaerosolspray, Haarwachs oder in Form eines emulsionsförmigen Haarpflegemittels (Haarspülung, Conditioner) appliziert werden. Das Mittel kann auch als Shampoo in Kombination mit anionischen, amphoteren, nichtionischen oder kationischen Tensiden oder als Färbemittel in Kombination mit direktziehenden oder oxidativen Haarfarben, z.B. als Farbfestiger vorliegen. Besonders bevorzugt ist die Anwendung als nicht auszuspülendes Leave-in-Produkt.

Wenn das erfindungsgemäße Haarbehandlungsmittel in Form eines Aerosolsprays vorliegt, so enthält es zusätzlich 15 bis 85 Gewichtsprozent, bevorzugt 25 bis 75 Gewichtsprozent eines Treibmittels und wird in einem Druckbehälter abgefüllt. Als Treibmittel sind beispielsweise niedere Alkane, wie zum Beispiel n-Butan, i-Butan und Propan, oder auch deren Gemische sowie Dimethylether oder Fluorkohlenwasserstoffe wie F 152a (1,1-Difluorethan) oder F 134 (Tetrafluorethan) sowie ferner bei den in Betracht kommenden Drücken gasförmig vorliegende Treibmittel, wie beispielsweise N₂, N₂O und CO₂ sowie Gemische der vorstehend genannten Treibmittel geeignet.

Wenn das erfindungsgemäße Haarbehandlungsmittel in Form eines versprühbaren Non-Aerosol-Haarsprays vorliegt, so wird es mit Hilfe einer geeigneten mechanisch betriebenen Sprühvorrichtung versprüht. Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Wenn das erfindungsgemäße Haarbehandlungsmittel in Form eines Haarschaumes (Mousse) vorliegt, so enthält es mindestens eine übliche, hierfür bekannte schaumgebende Substanz. Das Mittel wird mit oder ohne Hilfe von Treibgasen oder chemischen Treibmitteln verschäumt und als Schaum in das Haar eingearbeitet und ohne Ausspülen im Haar belassen. Das erfindungsgemäße Mittel weist als zusätzliche Komponente eine Vorrichtung zum Verschäumen der Zusammensetzung auf. Unter Vorrichtungen zum Verschäumenen sind solche Vorrichtungen zu verstehen, welche das Verschäumen einer Flüssigkeit mit oder ohne Verwendung eines Treibmittels ermöglichen. Als geeignete mechanische Schäumvorrichtung kann beispielsweise ein handelsüblicher Pumpschäumer oder ein Aerosolschaumkopf verwendet werden.

Wenn das erfindungsgemäße Haarbehandlungsmittel in Form eines Haargels vorliegt, so enthält es zusätzlich mindestens eine gelbildende Substanz, beispielsweise eines der oben aufgeführten Verdickungsmittel in einer Menge von vorzugsweise 0,05 bis 10, besonders bevorzugt von 0,1 bis 2 Gew.%. Die Viskosität des Gels beträgt vorzugsweise von 500 bis 50.000 cSt, besonders bevorzugt von 1.000 bis 15.000 cSt bei 25°C (gemessen mit einem Rotationsviskosimeter RheoStress 100 der Firma Haake bei einer Temperatur von 25°C und einem Schergefälle von 0,5 bis 1400 s⁻¹.

Wenn das erfindungsgemäße Haarbehandlungsmittel in Form eines Haarwachses vorliegt, so enthält es zusätzlich wasserunlösliche Fett- oder Wachsstoffe oder Stoffe, die der Zusammensetzung eine wachsähnliche Konsistenz verleihen, in einer Menge von vorzugsweise 0,5 bis 30 Gewichtsprozent. Geeignete wasserunlösliche Stoffe sind beispielsweise Emulgatoren mit einem HLB-Wert unterhalb von 7, Silikonöle, Silikonwachse, Wachse (z.B. Wachsalkohole, Wachssäuren, Wachsester, sowie insbesondere natürliche Wachse wie Bienenwachs, Carnaubawachs, etc.), Fettalkohole, Fettsäuren, Fettsäureester oder hochmolekulare Polyethylenglykole mit einem Molekulargewicht von 800 bis 20.000, vorzugsweise von 2.000 bis 10.000 g/mol.

Wenn das erfindungsgemäße Haarbehandlungsmittel in Form einer Haarlotion vorliegt, so liegt es als im wesentlichen nicht-viskose oder gering viskose, fließfähige Lösung, Dispersion oder Emulsion mit einem Gehalt an mindestens 10 Gewichtsprozent, vorzugsweise 20 bis 95 Gewichtsprozent eines kosmetisch verträglichen Alkohols vor. Als Alkohole können insbesondere die für kosmetische Zwecke üblicherweise verwendeten niederen Alkohole mit 1 bis 4 Kohlenstoffatomen wie zum Beispiel Ethanol und Isopropanol verwendet werden.

Wenn das erfindungsgemäße Haarbehandlungsmittel in Form einer Haarcreme vorliegt, so liegt es vorzugsweise als Emulsion vor und enthält entweder zusätzlich viskositätsgebende Inhaltastoffe wie die oben genannten Verdicker in einer Menge von 0,1 bis 10 Gewichtsprozent oder die erforderliche Viskosität und cremige Konsistenz wird durch Micellbildung mit Hilfe von geeigneten Emulgatoren, Fettsäuren, Fettalkoholen, Wachsen etc. in üblicher Weise aufgebaut.

Wird das erfindungsgemäße kosmetische Mittel zur Haarpflege oder -festigung eingesetzt, so wird es folgendermaßen angewandt: Nach der Haarwäsche werden in dem handtuchtrockenen Haar, je nach Haarfülle, 5 bis 30 g des Mittels verteilt. Anschließend wird das Haar durchgekämmt und zur Frisur geformt und getrocknet.

Wenn das erfindungsgemäße Mittel als Mittel zur dauerhaften Verformung des Haares vorliegt, enthält es zusätzlich 0,5 bis 15 Gewichtsprozent mindestens einer keratinreduzierenden Mercaptoverbindung und liegt bevorzugt als wässrige, alkalisch oder schwach sauer (pH 5 bis 10) eingestellte Zubereitung vor, welche als keratinreduzierende Mercaptoverbindung beispielsweise Cystein, Cysteamin, N-Acetyl-L-Cystein, Mercaptocarbonsäure, wie zum Beispiel Thioglykolsäure oder Thiomilchsäure, oder Salze von Mercaptocarbonsäuren, wie zum Beispiel Ammonium- und Guanidinsalze der Thioglykolsäure oder Thiomilchsäure enthält.

Wird das erfindungsgemäße Mittel in Form eines Haartönungsmittels eingesetzt, so enthält es zusätzlich 0,05 bis 2,0 Gewichtsprozent mindestens eines direkt auf das Haar aufziehenden Haarfarbstoffs, der beispielsweise aus den folgenden Klassen direkt auf das Haar aufziehender Haarfarbstoffe ausgewählt sein kann: aromatische Nitrofarbstoffe, zum Beispiel 1,4-Diamino-2-nitrobenzol, Azofarbstoffe, zum Beispiel Acid Brown 4 (C. I. 14 805), Anthrachinonfarbstoffe, zum Beispiel Disperse Voilet 4 (C. I. 61 105), Triphenylmethanfarbstoffe, zum Beispiel Basic Violet 1 (C. I. 42 535), wobei die Farbstoffe je nach Art ihrer Substituenten sauren, nichtionischen oder basischen Charakter haben können und/oder natürliche Haarfarbstoffe, wie zum Beispiel Henna oder Reng, der zur Farbentwicklung nicht der Oxidation bedarf.

Die folgenden Beispiele sollen die Gegenstände der vorliegenden Erfindung näher erläutern, ohne daß die angefügten Ansprüche hierauf beschränkt sind.

### Beispiele

Die in dieser Anmeldung angegebenen Viskositäten sind dynamische Viskositäten und werden unter folgenden Bedingungen gemessen:
Messgerät:
   Rotationsviskosimeter RheoStress 100 der Firma Haake
Temperatur: 25°C
Schergefälle: 0,5 bis 1400 s⁻¹

### Beispiel 1: Herstellung eines Saccharidlatex

Gemäß dem nachstehend angegebenen Rezept bzw. Batch-Verfahren wurde ein Saccharidlatex hergestellt.

| | |
|---|---|
| Wasser | 460,00 g |
| Natriumdodecylsulfat (NDS) | 10,00 g |
| Methacroyldiacetonglucose (MDG) | 143,80 g |
| n-Butylacrylat (BA) | 56,20 g |
| Kaliumpersulfat | 1,05 g |

In einem mit Rückflußkühler und Rührer versehenen Doppelwandreaktor wurde unter Rühren mit 200 U/min und unter Argon oder Stickstoff Atmosphäre 460 ml entgastes, bidestilliertes Wasser in den Reaktor eingefüllt. Der Reaktor wurde auf 60°C erwärmt. 10,00 g NDS werden zugegeben und gelöst. Dann werden 143,80 g MDG und 56,20 g n-Butylacrylat (BA) zugegeben. Nach ca. 10 Minuten Temperierzeit wird 1,05 g Kaliumperoxodisulfat (wasserlöslicher Initiator) addiert. Der Reaktor wird verschlossen und unter Rühren die Reaktion gestartet. Nach 4 bzw. 6 Stunden wird die Reaktion dann 15 min lang auf 70°C gehalten und auf Raumtemperatur abgekühlt. Der pH des Dispersionslatex wurde durch Zugabe von 25%iger wässriger Ammoniaklösung auf 7 bis 8 eingestellt. Der erhaltene Saccharidlatex wies die folgenden physikalischen Eigenschaften auf: Feststoffgehalt: 29,7 %; Teilchengröße: 60-65 nm; pH: 7,0; Viskosität: ca. 100-120 mPa.s.

### Beispiel 2: Herstellung eines Saccharidlatex nach dem Prä-emulsionsverfahren

| | |
|---|---|
| Wasser | 300,0 g |
| Natriumalkylphenolpolyglykolethersulfat, 35%ig (Rewopol® NOS10, WITCO GmbH) | 9,0 g |
| Kaliumpersulfat | 0,6 g |

| Monomermischung: | |
|---|---|
| Wasser | 300,0 g |
| Rewopol® NOS10 | 42,0 g |
| Methacroyldiacetonglucose (MDG) | 206,0 g |
| Butylacrylat (BA) | 400,0 g |
| Acrylsäure (AA) | 6,0 g |

| Initiatorlösung: | |
|---|---|
| Wasser | 150,0 g |
| Kaliumpersulfat | 2,1 g |

In einem mit Rückflußkühler, Tropftrichtern und Rührer versehenen 2 l Doppelwandreaktor wurde unter Rühren mit 200 U/min eine Anfangscharge von 300,0 g Wasser 9,00 g Rewopol NOS10 in den Reaktor eingefüllt. Der Reaktor wurde auf 60 °C erwärmt, dann wurde 0,60 g Kaliumpersulfat addiert. Die Monomermischung wurde kräftig vermischt und danach in das Reaktionsgefäß über eine Zeitspanne von 3 Stunden dosiert. Die Initiatorlösung wurde über eine Zeitspanne von 3 Stunden 30 min in den Reaktor dosiert. Die Reaktion wurde dann 45 min lang auf 70°C gehalten und auf Raumtemperatur abgekühlt. Der pH des Dispersionslatex wurde durch Zugabe von 25%iger wässriger Ammoniaklösung auf 7 bis 8 eingestellt. Der erhaltene Saccharidlatex wies die folgenden physikalischen Eigenschaften auf: Feststoffgehalt: 44-45%; Teilchengröße: 100 nm; pH: 8,0; Viskosität: ca. 100 mPa.s.

### Beispiel 3: Herstellung eines Saccharidlatex nach dem Shot-Growth-Verfahren

| | |
|---|---|
| Wasser | 390,00 g |
| Natriumdodecylsulfat (NDS) | 4,00 g |
| Butylacrylat | 112,00 g |
| Styrol | 48,00 g |
| Kaliumpersulfat | 0,76 g |

| Monomerzugabe: | |
|---|---|
| Methacroyldiacetonglucose (MDG) | 8,50 g |

In einem mit Rückflußkühler und Rührer versehenen Doppelwandreaktor wurde unter Rühren mit 200 U/min und unter Argon- oder Stickstoff-atmosphäre 390 ml entgastes, bidestilliertes Wasser in den Reaktor eingefüllt. Der Reaktor wurde auf 70 °C erwärmt. 4,00 g NDS werden zugegeben und gelöst. Dann wird eine Monomermischung aus 48,00 g Styrol und 112,00 g n-Butylacrylat zugegeben. Nach ca. 10 min Temperierzeit wird 0,46 g Kaliumperoxodisulfat addiert. Der Reaktor wird verschlossen und unter Rühren die Reaktion gestartet. Nach 2 Stunden Reaktionszeit (Umsatz ca. 80-90%) werden 8,50 g MDG auf einmal zugeführt. Nach 4 Stunden wird die Reaktion 20 min lang auf 80°C gehalten, dann auf Raumtemperatur abgekühlt. Der pH des Dispersionslatex wurde durch Zugabe von 25%iger wässriger Ammoniaklösung auf 7 bis 8 eingestellt. Der erhaltene Saccharidlatex wies die folgenden physikalischen Eigenschaften auf: Feststoffgehalt: 29,8 %; Teilchengröße: 79 nm; pH: 7,8; Viskosität: ca. 90 mPa.s.

### Beispiel 4: Herstellung von Saccharidlatex mit Kern-Schale-Polymerpartikeln im 2-stufigen Verfahren

| | |
|---|---|
| Wasser | 400,00 g |
| Natriumdodecylsulfat (NDS) | 8,40 g |
| Kaliumpersulfat | 0,56 g |

| Monomermischung: | Feed1 (Kern) | Feed2(Schale) |
|---|---|---|
| Wasser | 320,00 g | 160,00 g |
| Natriumdodecylsulfat(NDS) | 33,60 g | 1,12 g |
| Methylmethacrylat (MMA) | 205,85 g | - |
| Butylacrylat (BA) | 336,00 g | - |
| Methacroyldiacetonglucose (MDG) | - | 120,00 g |
| Methacrylsäure (MAA) | 2,25 g | - |

| Initiatorlösung: | Cofeed1 | Cofeed2 |
|---|---|---|
| Wasser | 100,00 g | 20,00 g |
| Kaliumpersulfat | 2,18 g | 0,61 g |

In einem mit Rückflußkühler, Tropftrichtern und Rührer versehener 2 l Doppelwandreaktor wurde unter Rühren eine Anfangscharge von 400 g Wasser und 8,40 g NDS in den Reaktor eingefüllt. Der Reaktor wurde auf 60 °C unter Stickstoff erwärmt. Die Monomermischung Feed1 wurde kräftig vermischt und ein Teil davon (ca. 10 Gew.%) sowie im Anschluß hieran 0,56 g Kaliumpersulfat in das Reaktionsgefäß addiert. Das Reaktionsgemisch wurde 30 Minuten lang auf 60 °C gehalten. Der Rest von Feed1 wurde über eine Zeitspanne von 2 Stunden dosiert. Gleichzeitig wurde die Initiatorlösung Cofeed 1 über eine Zeitspanne von 2 Stunden 30 min in den Reaktor dosiert. Die Reaktion wurde dann 20 min lang auf 70°C gehalten und auf 60 °C wiederabgekühlt. Dann wurde die Monomermischung Feed2 langsam über eine Zeitspanne von 90 Minuten dosiert, während die Initiatorlösung Cofeed 2 über eine Zeitspanne von 110 Minuten addiert wurde. Der pH des Dispersionslatex wurde durch Zugabe von 25%iger wässriger Ammoniaklösung auf 7 bis 8 eingestellt. Der erhaltene Saccharidlatex wies die folgenden physikalischen Eigenschaften auf: Feststoffgehalt: 40 %; Teilchengröße: 144 nm; pH: 8,0; Viskosität: ca. 85 mPa.s.

### Beispiel 5: Herstellung von Kern-Schale Saccharidlatex mit multifunktionellen Verbindungen

Beispiel 4 wurde mit der Abänderung wiederholt, daß man zusätzlich 18,15 g der multifunktionellen Verbindung Allylmethacrylat (ALMA) in Feed 1 zur Bildung des Kerns einsetzte. In der Initiatorlösung wurde die Menge Kaliumpersulfats auf 2,29 g erhöht. Man erhielt einen Saccharidlatex, der folgende physikalische Eigenschaften aufweist:
Feststoffgehalt: 40 %; Teilchengröße: 225 nm; pH: 8,0; Viskosität: ca. 55-60 mPa.s.

### Beispiel 6: Herstellung eines Kern-Schale-Latex mit multifunktionellen Verbindungen und Reglern

Beispiel 5 wurde mit der Abänderung wiederholt, daß man zusätzlich 6,79 g n-Dodecylmercaptan als Regler in Feed 2 und die folgende Monomermischung einsetzte:

| Monomermischung: | Feed1 (Kern) | Feed2(Schale) |
|---|---|---|
| Wasser | 320,00 g | 160,00 g |
| Natriumdodecylsulfat(NDS) | 33,60 g | 1,12 g |
| Methylmethacrylat (MMA) | 249,88 g | - |
| Butylacrylat (BA) | 292,37 g | - |
| Methacroyldiacetonglucose (MDG) | - | 120,00 g |
| Methacrylsäure (MAA) | 2,25 g | - |
| Allylmethacrylat (ALMA) | 18,15 g | - |
| n-Dodecylmercaptan | - | 6,79 g |

Man erhielt einen Saccharidlatex, der folgende physikalische Eigenschaften aufweist: Feststoffgehalt: 40 %; Teilchengröße: 225 nm; pH: 8,0; Viskosität: ca. 60-65 mPa.s.

### Kosmetische Mittel

In den Beispielen für kosmetische Mittel wurden die in Tabelle 1 aufgeführten Saccharid-Latices mit den in Tabelle 2 aufgeführten Eigenschaften verwendet. Die Latices wurden wie in Beispiel 1 mit den folgenden Parametern hergestellt:

**Tabelle 1**

| | MDG:BA (Mol%) | Tensid (Gew.%) | Initiator (Mol%) | Temperatur (°C) | Zeit (min) | Umsatz (%) |
|---|---|---|---|---|---|---|
| Latex 1 | 59:41 | 1 | 0,4 | 60 | 240 | 86 |
| Latex 2 | 37:63 | 2 | 0,4 | 70 | 270 | 95 |
| Latex 3 | 37:63 | 1 | 0,4 | 70 | 220 | 98 |
| Latex 4 | 59:41 | 0,5 | 0,4 | 70 | 345 | 97 |
| Latex 5 | 37:63 | 1,2 | 0,4 | 70 | 300 | 98 |
| MDG: Methacryloyldiacetonglucose, BA: n-Butylacrylat, Tensid: Natriumdodecylsulfat, Initiator: K₂S₂O₈ | | | | | | |

**Tabelle 2**

| | Trockensubstanz (%) | Teilchengröße (nm) | Viskosität (mPa.s) | Polymerzusammensetzung¹⁾ | Tg (°C) |
|---|---|---|---|---|---|
| Latex 1 | 8,2 | 51 | 1,2 | 49:51 | 132 |
| Latex 2 | 16,5 | 56 | 1,97 | 38:62 | 65 |
| Latex 3 | 18 | 63 | 2,1 | 39:61 | 107 |
| Latex 4 | 9 | 65 | 1,2 | 51:49 | 68 |
| Latex 5 | 35 | 63 | 3,88 | 31:69 | 62 |

| | | | | | |
|---|---|---|---|---|---|
| ¹⁾MDG:BA gemäß ¹H-NMR | | | | | |

Die in den folgenden Beispielen genannten Mengenangaben für die Latices beziehen sich jeweils auf den Feststoffgehalt.

### Beispiel 7: Haarfestigungsmittel

- 3,10 g: Latex 2
- 0,20 g: 1,2-Propylenglykol
- 0,15 g: Parfüm
- ad 100 g: Wasser

### Beispiel 8: Haarfestigungsmittel

- 3,10 g: Latex 3
- 0,20 g: 1,2-Propylenglykol
- 0,15 g: Parfüm
- ad 100 g: Wasser

### Beispiel 9: Haarfestigungsmittel

- 3,10 g: Latex 5
- 0,20 g: 1,2-Propylenglykol
- 0,15 g: Parfüm
- ad 100 g: Wasser

### Beispiel 10: Schaumfestiger

- 1,25 g: Latex 2
- 2,15 g: Polyvinylpyrrolidon
- 0,20 g: Zitronensäure
- 0,60 g: Hydriertes Rizinusöl,
ethoxyliert mit 4 Mol Ethylenoxid
- 0,20 g: Vinylpyrrolidon/Methacrylamidopropyl trimethylammoniumchlorid Copolymer
- 0,20 g: Parfüm
- 6,00 g: Propan/Butan (5,0 bar)
- ad 100 g: Wasser

### Beispiel 11: Schaumfestiger

- 3,25 g: Latex 4
- 0,15 g: Zitronensäure
- 0,60 g: Hydriertes Rizinusöl,
ethoxyliert mit 4 Mol Ethylenoxid
- 0,20 g: Vinylpyrrolidon/Methacrylamidopropyltrimethylammoniumchlorid Copolymer
- 0,20 g: Parfüm
- 6,00 g: Propan/Butan (5,0 bar)
- ad 100 g: Wasser

### Beispiel 12: Schaumfestiger

- 1,25 g: Latex 2
- 1,15 g: Polyvinylpyrrolidon
- 0,20 g: Zitronensäure
- 0,60 g: Hydriertes Rizinusöl,
ethoxyliert mit 4 Mol Ethylenoxid
- 0,20 g: Parfüm
- 6,00 g: Propan/Butan (5,0 bar)
- ad 100 g: Wasser

### Beispiel 13: Haarspray

- 3,55 g: Latex 2
- 0,15 g: Parfüm
- 0,14 g: Ameisensäure
- 45,00 g: Dimethylether
- ad 100 g: Ethanol

### Beispiel 14: 80% VOC-Haarspray

- 2,10 g: Latex 2
- 2,00 g: t-Butylacrylat/Ethylacrylat/Methacrylsäure Terpolymer
- 0,72 g: 2-Amino-2-methyl-1-propanol
- 0,20 g: Cyclo-Tetra(dimethylsiloxan)
- 0,05 g: Parfüm
- 15,00 g: Wasser
- 40,00 g: Dimethylether
- ad 100 g: Ethanol

### Beispiel 15: 80% VOC-Haarspray

- 2,10 g: Latex 3
- 2,00 g: t-Butylacrylat/Ethylacrylat/Methacrylsäure Terpolymer
- 0,72 g: 2-Amino-2-methyl-1-propanol
- 0,20 g: Cyclo-Tetra(dimethylsiloxan)
- 0,05 g: Parfüm
- 15,00 g: Wasser
- 40,00 g: Dimethylether
- ad 100 g: Ethanol

### Beispiel 16: 55% VOC-Pumpspray

- 4,10 g: Latex 2
- 0,28 g: Ameisensäure
- 0,20 g: Parfüm
- 55,00g: Ethanol
- ad 100 g: Wasser

### Beispiel 17: Haargel

- 0,50 g: Latex 1
- 2,50 g: Hydroxypropylmethylcellulose
- 0,80 g: Polyoxyethylen-(20)-sorbitanmonopalmitat
- 0,50 g: Polyoxyethylen-(25)-p-aminobenzoesäure
- 0,10 g: Parfüm
- 23,00 g: Glycerin (86prozentig)
- ad 100 g: Wasser

### Beispiel 18: Farbfestiger

- 0,23 g: Latex 2
- 2,50 g: Vinylacetat/Crotonsäure/Polyglykol Copolymer
- 0,20 g: Parfüm
- 0,07 g: 1-Amino-4-(2*'*,3*'*-dehydroxypropyl)amino-5-chlor-2-nitrobenzol
- 0,05 g: Basic Brown 17 (C. I. 12 251)
- 0,01 g: Basic Blue 7 (C. I. 42 595)
- 0,0023 g: Basic Violett 14 (C. I. 42 510)
- 50,00 g: Ethanol
- ad 100 g: Wasser

## Patentansprüche

1. Kosmetisches Mittel mit einem Gehalt an mindestens einem Polymer, welches aus mindestens einer Monomerart (A) aufgebaut ist, welche ein mit mindestens einer radikalisch polymerisierbaren Gruppe substituiertes Kohlenhydrat oder Kohlenhydratderivat ist und eine oder mehrere Schutzgruppen enthalten kann in einer geeigneten kosmetischen Grundlage.

2. Mittel nach Anspruch 1, dadurch gekennzeichnet, daß das Polymer zusätzlich zu Monomerart (A) aufgebaut ist aus mindestens einer zweiten, von (A) verschiedenen, mit (A) radikalisch copolymerisierbaren Monomerart (B).

3. Mittel nach Anspruch 1 oder 2, dadurch gekennzeichnet, daß das Polymer in wässrigem Medium unlöslich ist und in Form eines Polymerlatex eingesetzt wird.

4. Mittel nach Anspruch 3, dadurch gekennzeichnet, daß das Polymerlatex mindestens ein in Wasser dispergiertes, durch Emulsionspolymerisation hergestelltes Polymer enthält, welches aufgebaut ist aus
(A) mindestens einer ersten Monomerart, welche ein mit mindestens einer radikalisch polymerisierbaren Gruppe substituiertes Kohlehydrat oder Kohlehydratderivat ist, wobei die Monomerart eine oder mehrere Schutzgruppen enthalten kann und
(B) mindestens einer zweiten, von (A) verschiedenen, mit (A) radikalisch copolymerisierbaren Monomerart.

5. Mittel nach Anspruch 4, dadurch gekennzeichnet, daß das die Monomerart (A) enthaltende Polymer die allgemeine Formel (I) aufweist
-[CH₂-C(Y)CO-D-A]ₘ-[B]ₙ- (I)
wobei
Y für R, CH2COOR oder COOR und R für H oder für einen C1- bis C18-Kohlenwasserstoffrest steht,
D für Sauerstoff oder Stickstoff steht,
A für ein Saccharid, ein Saccharidderivat oder für einen Zuckeralkohol steht und eine oder mehrere Schutzgruppen enthalten kann,
B für ein radikalisch polymerisierbares Monomer steht, welches keine Gruppe A enthält und
m und n für Zahlen stehen, die den jeweiligen Monomergehalt angeben, wobei m größer Null und n größer oder gleich Null ist.

6. Mittel nach Anspruch 5, dadurch gekennzeichnet, daß das Comonomer B ein Monomer oder eine Monomermischung ist, ausgewählt aus den Gruppen
(a) monoethylenisch ungesättigte C3- bis C12-Carbonsäuren oder deren Salze,
(b) monoethylenisch ungesättigte Ester von C3- bis C12-Carbonsäuren und C1- bis C14-Alkoholen,
(c) Acrylsäure- oder Methacrylsäuredialkylaminoalkylester mit insgesamt bis zu 30 C-Atomen im Dialkylaminoalkyl-Rest, welche in N-quaternisierter Form oder in Salzform vorliegen können,
(d) Amide der unter (a) genannten ungesättigten Carbonsäuren, z.B. Acrylsäureamid, Methacrylsäureamid, N,N-Dialkyl-acrylsäure- oder -methacrylsäureamid,
(e) fünf- bis achtgliedrige N-Vinyllactame, welche am Ring durch bis zu drei C1- bis C12-Alkylreste substituiert sein können,
(f) Maleinsäure- und Fumarsäuredialkylester mit bis zu 12 C-Atomen pro Alkylrest,
(g) monoethylenisch ungesättigte C3- bis C12-Alkylvinylether,
(h) Vinylaromaten, wie z.B. Styrol, welches am aromatischen Ring durch ein bis drei C1- bis C12-Alkylreste substituiert sein kann,
(i) ein- oder mehrfach ungesättigte Kohlenwasserstoffe, wie z.B. Ethylen, Propylen oder 1,3-Butadien,
(j) Vinylhalogenide, wie z.B. Vinylchlorid.

7. Mittel nach einem der vorstehenden Ansprüche, dadurch gekennzeichnet, daß die Monomerart (A) bzw. der Rest A abgeleitet ist von Glucose, Fructose, Galactose, Sorbose oder Xylit und eine oder mehrere Schutzgruppen enthalten kann.

8. Mittel nach einem der vorstehenden Ansprüche, dadurch gekennzeichnet, daß die Schutzgruppe ausgewählt ist aus Isopropyliden- oder Cyclohexyliden-Schutzgruppen.

9. Mittel nach einem der Ansprüche 5 bis 8, dadurch gekennzeichnet, daß die Gruppe Y ausgewählt ist aus Wasserstoff, Methyl, -COOH, -COOCH₃ und - COOC₂H₅.

10. Mittel nach einem der Ansprüche 2 bis 9, dadurch gekennzeichnet, daß das Verhältnis von Monomerart (A) zu Monomerart (B) von 2:98 bis 98:2, vorzugsweise von 30:70 bis 70:30 beträgt.

11. Mittel nach einem der vorgenannten Ansprüche, dadurch gekennzeichnet, daß die Monomerart (A) die allgemeine Formel (III) aufweist 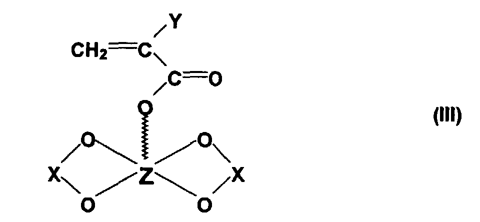 wobei Z einen Glucose- oder Fructoserest, X eine Isopropyliden- oder eine Cyclohexylidengruppe und Y Wasserstoff oder Methyl bedeuten.

12. Mittel nach Anspruch 3, dadurch gekennzeichnet, daß das Polymerlatex in Wasser dispergierte Komposit-Polymerpartikel, insbesondere Kern/Schale-Polymerpartikel, enthält, welche durch mindestens zweistufige Emulsionspolymerisation hergestellt sind und welche aufgebaut sind aus mindestens einem Polymer, welches seinerseits aufgebaut ist aus mindestens einer Monomerart (A), welche ein mit mindestens einer radikalisch polymerisierbaren Gruppe substituiertes Kohlenhydrat oder Kohlenhydratderivat ist, wobei die Monomerart (A) eine oder mehrere Schutzgruppen enthalten kann.

13. Mittel nach Anspruch 12, dadurch gekennzeichnet, daß das Polymerisat der zweiten Polymerisationsstufe (Schale) mindestens ein Polymer enthält, welches die Monomerart (A) enthält und das Polymerisat der ersten Polymerisationsstufe (Kern) aus einem beliebigen Polymer aufgebaut ist.

14. Mittel nach Anspruch 13, dadurch gekennzeichnet, daß das Polymerisat der ersten Polymerisationsstufe (Kern) keine Bestandteile der Monomerart (A) enthält.

15. Mittel nach Anspruch 12, dadurch gekennzeichnet, daß das Polymerisat der ersten Polymerisationsstufe (Kern) mindestens ein Polymer enthält, welches die Monomerart (A) enthält und das Polymerisat der zweiten Polymerisationsstufe (Schale) aus einem beliebigen Polymer aufgebaut ist.

16. Mittel nach Anspruch 15, dadurch gekennzeichnet, daß das Polymerisat der zweiten Polymerisationsstufe (Schale) keine Bestandteile der Monomerart (A) enthält.

17. Mittel nach einem der Ansprüche 12 bis 16, dadurch gekennzeichnet, daß das die Monomerart (A) enthaltende Polymer ein Homopolymer der allgemeinen Formel (II) ist
-[CH₂-C(Y)CO-D-A]ₘ- (II)
wobei
Y für R, CH2COOR oder COOR und R für H oder für einen C1- bis C18-Kohlenwasserstoffrest steht,
D für Sauerstoff oder Stickstoff steht,
A für ein Saccharid, ein Saccharidderivat oder für einen Zuckeralkohol steht und eine oder mehrere Schutzgruppen enthalten kann und
m für eine Zahl größer Null steht, die den Polymerisationsgrad angibt.

18. Mittel nach einem der Ansprüche 12 bis 17, dadurch gekennzeinet, daß der Durchmesser der Komposit-Polymerpartikel 40 bis 1000 nm beträgt.

19. Mittel nach einem der Ansprüche 12 bis 18, dadurch gekennzeichnet, daß der Gewichtsanteil des Polymerisats der zweiten Polymerisationsstufe (Schale) 3 bis 50% des Gesamtgewichts der Komposit-Polymerpartikel beträgt.

20. Mittel nach einem der vorgenannten Ansprüche, dadurch gekennzeichnet, daß das die Monomerart (A) enthaltende Polymer zu 0,01 bis 25 Gew.% enthalten ist.

21. Mittel nach einem der vorgenannten Ansprüche, dadurch gekennzeichnet, daß es ein zusätzliches, filmbildendes Polymer in einer Menge von vorzugsweise 0,01 bis 25 Gew.% enthält.

22. Mittel nach einem der vorgenannten Ansprüche, dadurch gekennzeichnet, daß es in Form einer Lotion, einer Sprühlotion, einer Creme, eines Gels, eines Aerosol- oder Nonaerosol-Sprays, eines Aerosol- oder Nonaerosol-Schaums, eines Shampoos, einer Mascara, eines Make-ups, eines Lippenstifts oder eines Körperdeodorans vorliegt.
